(19) Europäisches Patentamt
European Patent Office
Office européen des brevets

(11) Veröffentlichungsnummer: **0 277 537 B1**

# (12) EUROPÄISCHE PATENTSCHRIFT

(45) Veröffentlichungstag der Patentschrift: **26.09.90**

(51) Int. Cl.⁵: **C07D 207/34**, C07D 401/06, C07D 403/06, A01N 43/36

(21) Anmeldenummer: **88100642.3**

(22) Anmeldetag: **19.01.88**

(54) **1-Aminomethyl-3-aryl-4-cyano-pyrrole.**

(30) Priorität: **31.01.87 DE 3702853**

(43) Veröffentlichungstag der Anmeldung: **10.08.88 Patentblatt 88/32**

(45) Bekanntmachung des Hinweises auf die Patenterteilung: **26.09.90 Patentblatt 90/39**

(84) Benannte Vertragsstaaten: **AT BE CH DE ES FR GB GR IT LI NL**

(56) Entgegenhaltungen:
**EP-A- 0 133 247**
**EP-A- 0 174 910**
**EP-A- 0 182 737**
**EP-A- 0 182 738**
**EP-A- 0 206 999**
**US-A- 4 546 099**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Wollweber, Detlef, Dr., Kirschbaumstrasse 34, D-5600 Wuppertal 1(DE)**
Erfinder: **Brandes, Wilhelm, Dr., Eichendorffstrasse 3, D-5653 Leichlingen(DE)**
Erfinder: **Dutzmann, Stefan, Dr., Leinenweberweg 33, D-4000 Düsseldorf 13(DE)**
Erfinder: **Hänssler, Gerd, Dr., Am Arenzberg 58a, D-5090 Leverkusen 3(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 1-Aminomethyl-3-aryl-4-cyano-pyrrole, mehrere Verfahren zu ihrer Herstellung sowie ihre Verwendung als Schädlingsbekämpfungsmittel.

Es ist bereits bekannt, daß bestimmte Dithiocarbamate, wie beispielsweise das Zink-ethylen-1,2-bis-(dithiocarbamat) oder das Mangan-ethylen-1,2-bis-(dithiocarbamat) gute fungizide Eigenschaften besitzen (vgl. z.B. K.H. Büchel, "Pflanzenschutz und Schädlingsbekämpfung", S. 137, 138, Thieme Verlag, Stuttgart 1977). Weiterhin ist bekannt, daß bestimmte Sulfenamide, wie beispielsweise das N,N-Dimethyl-N'-phenyl-N'-(fluordichlormethylsulfenyl)-sulfamid insbesondere gegen Botrytis-Arten hervorragend wirksam sind (vgl. z.B. K.H. Büchel "Pflanzenschutz und Schädlingsbekämpfung", S. 141, Thieme Verlag Stuttgart 1977).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Weiterhin ist bekannt, daß bestimmte 3-Aryl-pyrrole, wie beispielsweise das 4-Cyano-3-(2,3-dichlorphenyl)-pyrrol ebenfalls gute fungizide Eigenschaften besitzen (vgl. z.B. EP 174 910 oder EP 182 738 oder EP 133 247).

Es wurden neue 1-Aminomethyl-3-aryl-4-cyano-pyrrole der allgemeinen Formel (I),

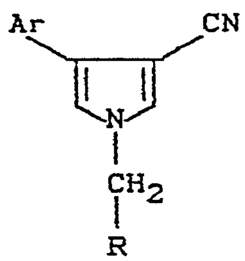

(I)

in welcher

Ar für gegebenenfalls einfach bis mehrfach, gleich oder verschieden substituiertes Phenyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

R für einen Rest 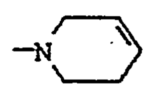 ; 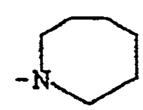 oder

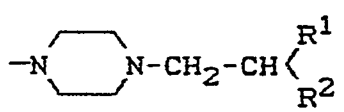 steht, wobei

$R^1$ für Wasserstoff oder für geradkettiges oder verzweigtes Alkyl mit 1 bis 6 Kohlenstoffatomen steht und

$R^2$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht, gefunden.

Weiterhin wurde gefunden, daß man die neuen 1-Aminomethyl-3-aryl-4-cyano-pyrrole der allgemeinen Formel (I),

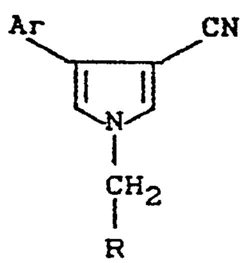

(I)

in welcher

Ar für gegebenenfalls einfach bis mehrfach, gleich oder verschieden substituiertes Phenyl steht, wobei

als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

R für einen Rest 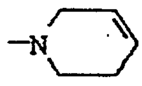 ; 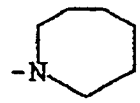 oder

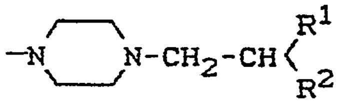 steht, wobei

$R^1$ für Wasserstoff oder für geradkettiges oder verzweigtes Alkyl mit 1 bis 6 Kohlenstoffatomen steht und
$R^2$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht, erhält, wenn man

(a) 3-Aryl-4-cyano-pyrrole der Formel (II),

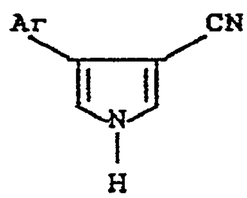 (II)

in welcher
Ar die oben angegebene Bedeutung hat, mit Formaldehyd und Aminen der Formel (III),
R–H (III)
in welcher
R die oben angegebene Bedeutung hat, gegebenenfalls in Gegenwart eines Verdünnungsmittels und gegebenenfalls in Gegenwart eines Reaktionshilfsmittels umsetzt, oder wenn man,

(b) 3-Aryl-4-cyano-pyrrole der Formel (IV),

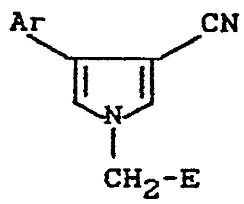 (IV)

in welcher
Ar die oben angegebene Bedeutung hat und
E für eine elektronenanziehende Abgangsgruppe steht, mit Aminen der Formel (III),
H–R (III)
in welcher
R die oben angegebene Bedeutung hat, gegebenenfalls in Gegenwart eines Verdünnungsmittels und gegebenenfalls in Gegenwart eines Säurebindemittels umsetzt.

Schließlicn wurde gefunden, daß die neuen 1-Aminomethyl-3-aryl-4-cyano-pyrrole der allgemeinen Formel (I) gute Wirkung gegen Schädlinge besitzen.

Überraschenderweise zeigen die erfindungsgemäßen 1-Aminomethyl-3-aryl-4-cyano-pyrrole der allgemeinen Formel (I) neben einer besseren fungiziden Wirksamkeit im Vergleich zu den aus dem Stand der Technik vorbekannten Dithiocarbamaten oder Sulfenamiden, wie beispielsweise das Zink-ethylen-1,2-bis-(dithiocarbamat) oder das Manganethylen-1,2-bis-(dithiocarbamat) oder N,N-Dimethyl-N'-phenyl-N'-(fluordichlormethylsulfenyl)-sulfamid gleichzeitig eine deutlich verbesserte Kulturpflanzenverträglichkeit im Vergleich zu den aus dem Stand der Technik vorbekannten 3-Aryl-pyrrolen, wie beispielsweise das 4-Cyano-3-(2,3-dichlorphenyl)-pyrrol, welches chemisch und/oder wirkungsmäßig naheliegende Verbindungen sind.

Die erfindungsgemäßen 1-Aminomethyl-3-aryl-4-cyanopyrrole sind durch die Formel (I) allgemein definiert. Bevorzugt sind Verbindungen der Formel (I), bei welchen
Ar für gegebenenfalls ein- bis dreifach, gleich oder verschieden substituiertes Phenyl steht, wobei als

Substituenten infrage kommen: Fluor, Chlor, Brom, Methyl, Ethyl, n- oder i-Propyl, n-, i-, s- oder t-Butyl, Methoxy, Ethoxy oder Methylthio, Trifluormethyl, Trifluormethoxy, Trifluormethylthio und

R für einen Rest 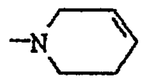 ; 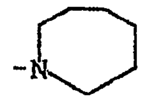 oder

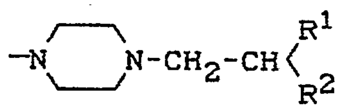 steht, wobei

$R^1$ für Wasserstoff, Methyl, Ethyl, n- oder i-Propyl oder n-, i-, s- oder t-Butyl steht und

$R^2$ für Cyano, Acetyl, Porpionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht.

Ganz besonders bevorzugt sind Verbindungen der Formel (I), bei welchen

Ar für einen 2,3-Dichlorphenylrest steht und

R für einen Rest 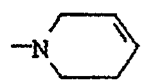 ; 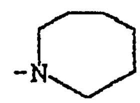 oder

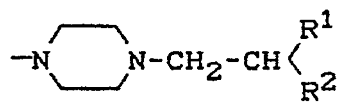 steht, wobei

$R^1$ für Wasserstoff steht und

$R^2$ für Cyano, Acetyl, Propionyl, Methoxycarbonyl, Ethoxycarbonyl, Dimethylaminocarbonyl, für Methylsulfinyl, Methylsulfonyl, Phenylsulfinyl oder Phenylsulfonyl steht.

Im einzelnen seien außer den bei den Herstellungsbeispielen genannten Verbindungen die folgenden 1-Aminomethyl-3-aryl-4-cyano-pyrrole der allgemeinen Formel (I) genannt:

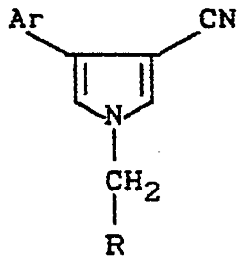

(I)

| Ar | R |
|---|---|
| 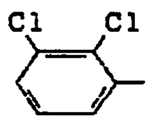 | 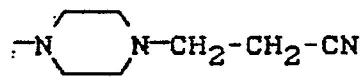 |
| 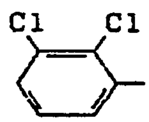 | 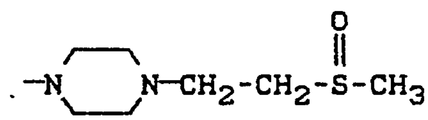 |

| Ar | R |
|---|---|
| 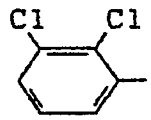 | 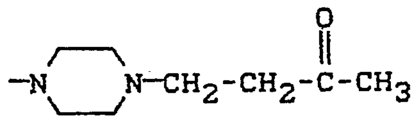 |
| 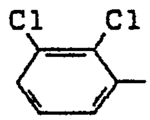 | 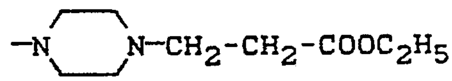 |
| 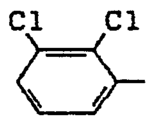 | 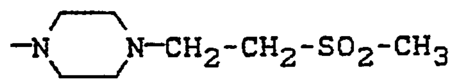 |
| 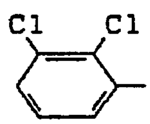 | 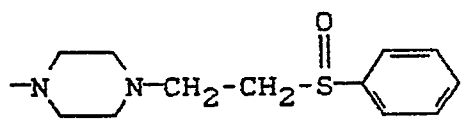 |
| 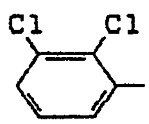 | 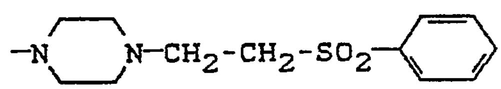 |
| 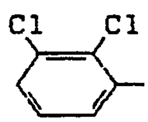 | 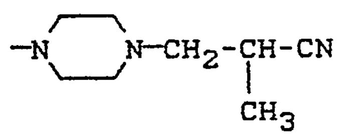 |

Verwendet man beispielsweise 4-Cyano-3-(2,3-dichlorphenyl)-pyrrol, Formaldehyd und Perhydroazepin als Ausgangsstoffe, so läßt sich der Reaktionsablauf des erfindungsgemäßen Verfahrens (a) durch das folgende Formelschema darstellen:

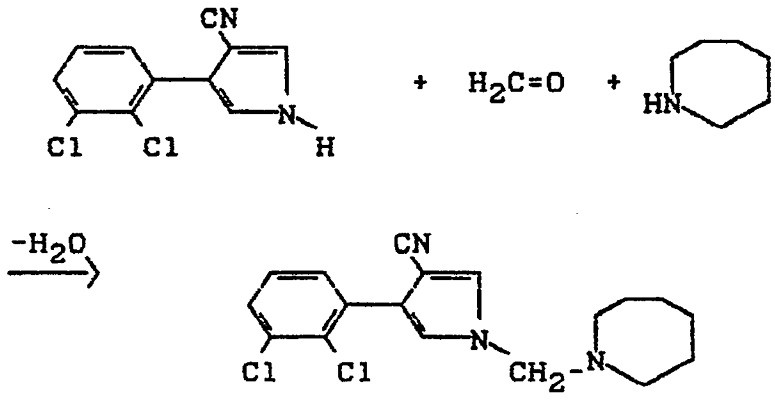


Verwendet man beispielsweise 1-Chlormethyl-4-cyano-3-(2,3-dichlorphenyl)-pyrrol und Tetrahydropyridin als Ausgangsverbindungen, so läßt sich der Reaktionsablauf des erfindungsgemäßen Verfahrens (b) durch das folgende Formelschema darstellen:

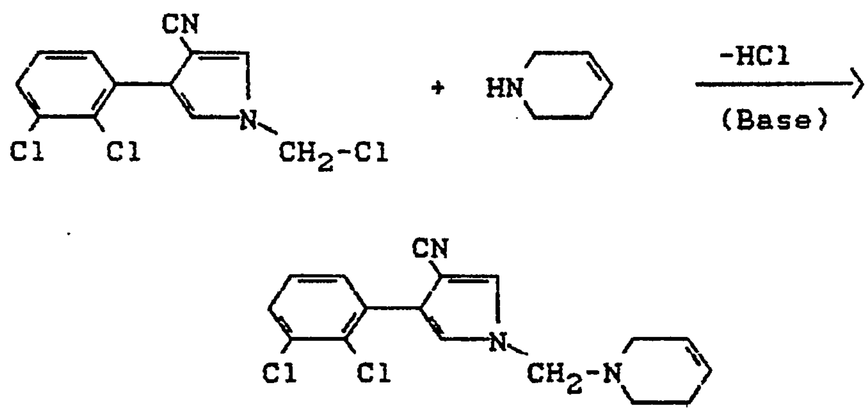


Die zur Durchführung des erfindungsgemäßen Verfahrens (a) als Ausgangsstoffe benötigten 3-Aryl-4-cyano-pyrrole sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die 3-Aryl-4-cyano-pyrrole der Formel (II) sind bekannt (vgl. z.B. EP 17 49 10, EO 182 738 oder EP 13 32 47).

Die zur Durchführung des erfindungsgemäßen Verfahrens (b) als Ausgangsstoffe benötigten 3-Aryl-4-cyano-pyrrole sind durch die Formel (IV) allgemein definiert. In dieser Formel (IV) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

E steht vorzugsweise für Hydroxy oder Halogen, insbesondere für Chlor.

Die 3-Aryl-4-cyano-pyrrole der Formel (IV) sind ebenfalls bekannt (vgl. z.B. EP 133 247).

Die zur Durchführung der erfindungsgemäßen Verfahren (a) und (b) weiterhin als Ausgangsstoffe benötigten Amine sind durch die Formel (III) allgemein definiert. In dieser Formel (III) steht R vorzugsweise für diejenigen Reste, die bereits im Zusammenhang mit der Beschreibung der erfindungsgemäßen Stoffe der Formel (I) als bevorzugt für diesen Substituenten genannt wurden.

Die Amine der Formel (III) sind allgemein bekannte Verbindungen der organischen Chemie oder erhältlich in Analogie zu allgemein bekannten Verfahren.

Als Verdünnungsmittel zur Durchführung des erfindungsgemäßen Verfahrens (a) kommen inerte organische Lösungsmittel oder wässrige System infrage.

Vorzugsweise verwendet man protische Lösungsmittel, beispielsweise Alkohole, wie Methanol, Ethanol oder Propanol, Carbonsäuren, wie Ameisensäure, Essigsäure oder Propionsäure oder deren Gemische mit Wasser. Es ist auch möglich, das erfindungsgemäße Verfahren (a) in aprotischen Lösungsmitteln durchzuführen.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphoshorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Reaktionshilfmittels durchgeführt. Hierzu eignen sich entweder katalytische bis äquimolare Mengen einer organischen oder anorganischen Säure oder entsprechende Mengen einer geeigneten Base.

Als saure Reaktionshilfsmittel kommen insbesondere anorganische Mineralsäuren, wie Phosphorsäure, Schwefelsäure, Salpetersäure, Chlorwasserstoffsäure oder Bromwasserstoffsäure oder organische Säuren, wie Ameisensäure, Essigsäure, Propionsäure, Methansulfonsäure, Benzolsulfonsäure oder Toluolsulfonsäure infrage.

Als basische Reaktionshilfsmittel kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich, das als Reaktionspartner verwendetete Amin der Formel (III) in entsprechendem Überschuß gleichzeitig als Reaktionshilfmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (a) setzt man pro Mol an 3-Aryl-4-cyanopyrrol der Formel (II) im allgemeinen 1,0 bis 2,0 Mol, vorzugsweise 1,0 bis 1,5 Mol an Amin der Formel (III) und 1,0 bis 2,0 Mol, vorzugsweise 1,0 bis 1,5 Mol an Formaldehyd ein. Der Formaldehyd wird entweder in Form einer wässrigen Lösung, als Paraformaldehyd oder als 1,3,5-Trioxan eingesetzt. Vorzugsweise verwendet man eine wässrige Lösung. Die Reaktionsdurchführung, Aufarbeitung und Isolierung der Reaktionsprodukte der Formel (I) erfolgt in Analogie zu bekannten Verfahren (vgl. EP 133 247).

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Buta non, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich das als Reaktionspartner in Frage kommende Amin der Formel (III) in entsprechendem Überschuß gleichzeitig als Reaktionshilfmittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (b) setzt man pro Mol an 3-Aryl-4-cyanopyrrol der Formel (IV) im allgemeinen 1,0 bis 2,0 Mol, vorzugsweise 1,0 bis 1,5 Mol an Amin der Formel (III) und gegebenenfalls 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (I) erfolgt in Analogie zu bekannten Verfahren.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch z.B. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;

Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides;
Sclerotinia-Arten, wie beispielsweise Sclerotinia sclerotiorum.

Die gute Pflanzenverträglichkeit der Wirkstoffe in den zur Bekämpfung von Pflanzenkrankheiten notwendigen Kon zentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Stengelgrundfäule des Weizens (Fusarium culmorum), zur Bekämpfung von Reiskrankheiten, wie beispielsweise gegen den Erreger der Reisfleckenkrankheit (Pyricularia oryzae) oder zur Bekämpfung von Krankheiten im Obst- und Gemüsebau, wie beispielsweise gegen den Erreger der Graufäule (Botrytis cinerea) einsetzen. Hervorzuheben ist dabei besonders, daß die erfindungsgemäßen Wirkstoffe neben einer guten protektiven Wirksamkeit auch systemische Eigenschaften besitzen und sich daher auch als Saatgutbeizmittel eignen. Hervorzuheben ist weiterhin eine gute und breite in vitro-Wirksamkeit der Verbindungen und eine gute Pflanzenverträglichkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cylcohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

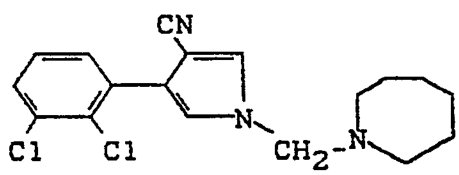

(Verfahren a)

Zu 4,0 g (0,017 Mol) 4-Cyano-3-(2,3-dichlorphenyl)-pyrrol (vgl. EP 133 247) in 17 ml Ethanol gibt man unter Rühren 1,6 g (0,02 Mol) einer 37prozentigen wässrigen Formaldehydlösung und 1,9 g (0,02 Mol) Hexamethylenimin. Man erhitzt so lange unter Rückfluß bis eine klare Lösung entsteht und rührt dann weitere 15 Stunden bei Raumtemperatur. Zur Aufarbeitung gibt man 100 ml Wasser zu, extrahiert dreimal mit jeweils 50 ml Essigester, trocknet die vereinigten organischen Phasen über Natriumsulfat und entfernt das Lösungsmittel im Vakuum. Das zurückbleibende Öl wird durch Verrühren mit heißem Petrolether gereinigt.

Man erhält 5,5 g (93 % der Theorie) an 4-Cyano-3-(2,3-dichlorphenyl)-1-(perhydroazepin-1-yl-methyl)-pyrrol als Öl. $^1$H-NMR ($CDCl_3$/TMS): $\delta$ = 4,8 ppm.

In entsprechender Weise und gemäß den allgemeinen Angaben zur Herstellung erhält man die folgenden 1-Amino-methyl-3-aryl-4-cyano-pyrrole der allgemeinen Formel (I):

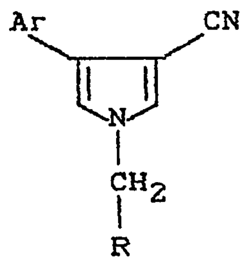

(I)

| Bsp. Nr. | Ar | R | physikalische Eigenschaften |
|---|---|---|---|
| 2 | 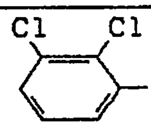 | 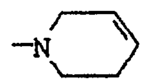 | $^{1}$H-NMR*): 4,8 |
| 3 | 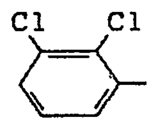 | 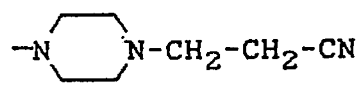 | Öl |

*) Die $^{1}$H-NMR-Spektren wurden in $CDCl_3$ mit Tetramethylsilan (TMS) als innerem Standard aufgenommen. Angegeben ist die chemische Verschiebung der

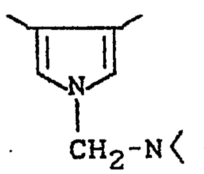

-Gruppe als δ-Wert in ppm.

## Anwendungsbeispiele

In den folgenden Anwendungsbeispielen wurden die nachstehend aufgeführten Verbindungen als Vergleichssubstanzen eingesetzt:

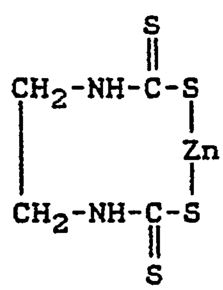

(A)

Zink-ethylen-1,2-bis-(dithiocarbamat)

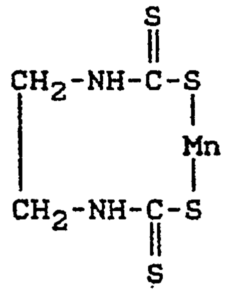

(B)

Mangan-ethylen-1,2-bis-(dithiocarbamat)

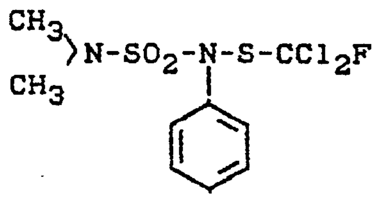

(C)

N,N-Dimethyl-N'-(fluordichlormethylsulfenyl)-sulfamid

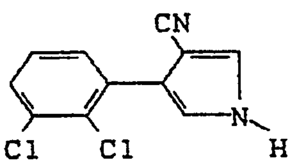

(D)

4-Cyano-3-(2,3-dichlorphenyl)-pyrrol
(bekannt aus EP 174 910).

Beispiel A

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

Eine deutliche Überlegenheit in der Wirksamkeit gegenüber dem Stand der Technik zeigen bei diesem Test z.B. die Verbindungen gemäß den Herstellungsbeispielen: 1 und 2.

Beispiel B

Botrytis-Test (Bohne)/protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 1 und 2.

Beispiel C

Pyricularia-Test (Reis) /protektiv
Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 1 und 2.

Beispiel D

Pflanzenverträglichkeitstest
Testpflanze: Reben
Versuchsdauer: 6 Tage
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Mit dieser Wirkstoffzubereitung bespritzt man junge Pflanzen bis zur Tropfnässe und stellt sie in einem Gewächshaus bei ca. 20 °C auf.

Die Pflanzen werden auf Schäden, wie Wuchsbeeinträchtigungen, Verfärbungen und Nekrosen ausgewertet. Nach den angegebenen Zeiten wird der Schädigungsgrad der Pflanzen bestimmt.

Eine deutliche Überlegenheit in der Pflanzenverträglichkeit gegenüber dem Stand der Technik zeigt bei diesem Test z.B. die Verbindung gemäß dem Herstellungsbeispiel: 1.

## Patentansprüche

1. 1-Aminomethyl-3-aryl-4-cyano-pyrrole der allgemeinen Formel (I),

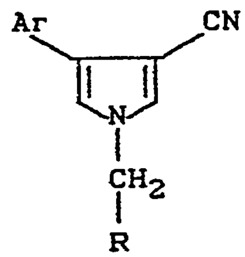

(I)

in welcher
Ar für gegebenenfalls einfach bis mehrfach, gleich oder verschieden substituiertes Phenyl steht, wobei als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

R für einen Rest 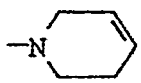 ; 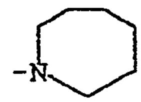 oder

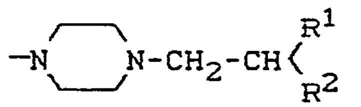 steht, wobei

$R^1$ für Wasserstoff oder für geradkettiges oder verzweigtes Alkyl mit 1 bis 6 Kohlenstoffatomen steht und

$R^2$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht.

2. 1-Aminomethyl-3-aryl-4-cyano-pyrrole gemäß Anspruch 1, wobei in der Formel (I)

Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy oder Methylthio, Trifluormethyl, Trifluormethoxy, Trifluormethylthio und

R für einen Rest 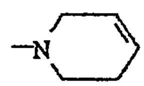 ; 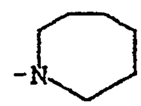 oder

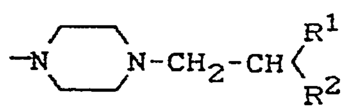 steht, wobei

$R^1$ für Wasserstoff, Methyl, Ethyl, n- oder i-Propyl oder n-, i-, s- oder t-Butyl steht und

$R^2$ für Cyano, Acetyl, Propionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht.

3. 1-Aminomethyl-3-aryl-4-cyano-pyrrole gemäß Anspruch 1, wobei in der Formel (I)

Ar für einen 2,3-Dichlorphenylrest steht und

R für einen Rest 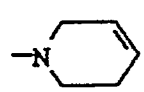 ; 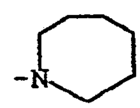 oder

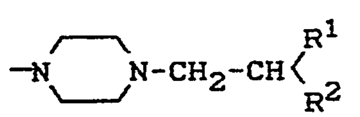 steht, wobei

$R^1$ für Wasserstoff steht und

$R^2$ für Cyano, Acetyl, Propionyl, Methoxycarbonyl, Ethoxycarbonyl, Dimethylaminocarbonyl, für Methylsulfinyl, Methylsulfonyl, Phenylsulfinyl oder Phenylsulfonyl steht.

4. Verfahren zur Herstellung von 1-Aminomethyl-3-aryl-4-cyano-pyrrolen der allgemeinen Formel (I)

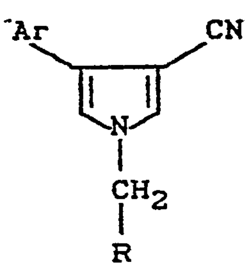 (I)

in welcher

Ar für gegebenenfalls einfach bis mehrfach, gleich oder verschieden substituiertes Phenyl steht, wobei

als Substituenten infrage kommen: Halogen, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen und

R für einen Rest 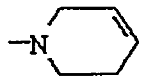 ; 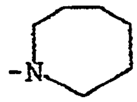 oder

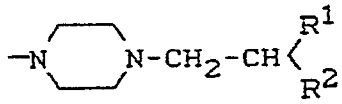 steht, wobei

$R^1$ für Wasserstoff oder für geradkettiges oder verzweigtes Alkyl mit 1 bis 6 Kohlenstoffatomen steht und
$R^2$ für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht, dadurch gekennzeichnet, daß man
(a) 3-Aryl-4-cyano-pyrrole der Formel (II),

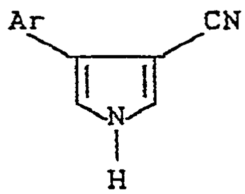 (II)

in welcher
Ar die oben angegebene Bedeutung hat,
mit Formaldehyd und Aminen der Formel (III),
R–H (III)
in welcher
R die oben angegebene Bedeutung hat, gegebenenfalls in Gegenwart eines Verdünnungsmittels und gegebenenfalls in Gegenwart eines Reaktionshilfsmittels umsetzt, oder
(b) 3-Aryl-4-cyano-pyrrole der Formel (IV),

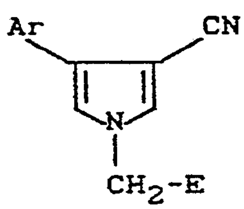 (IV)

in welcher
Ar die oben angegebene Bedeutung hat und
E für eine elektronenanziehende Abgangsgruppe steht, mit Aminen der Formel (III),
H–R (III)
in welcher
R die oben angegebene Bedeutung hat,gegebenenfalls in Gegenwart eines Verdünnungsmittels und gegebenenfalls in Gegenwart eines Säurebindemittels umsetzt.

5. Schädlingsbekämpfungsmittel, gekennzeichnet durch einen Gehalt an mindestens einem 1-Aminomethyl-3-aryl-4-cyano-pyrrol der Formel (I) nach den Ansprüchen 1 und 4.

6. Verwendung von 1-Aminomethyl-3-aryl-4-cyano-pyrrolen der Formel (I) nach den Ansprüchen 1 und 4 zur Bekämpfung von Schädlingen.

7. Verfahren zur Bekämpfung von Schädlingen, dadurch gekennzeichnet, daß man 1-Aminomethyl-3-aryl-4-cyano-pyrrole der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man 1-Aminomethyl-3-aryl-4-cyano-pyrrole der Formel (I) nach den Ansprüchen 1 und 4 mit Streckmitteln und/ oder oberflächenaktiven Mitteln vermischt.

9. Verwendung von 1-Aminomethyl-3-aryl-4-cyano-pyrrolen der Formel (I) gemäß Anspruch 5 zur Bekämpfung von Pilzen.

## Claims

1. 1-Aminomethyl-3-aryl-4-cyano-pyrroles of the general formula (I)

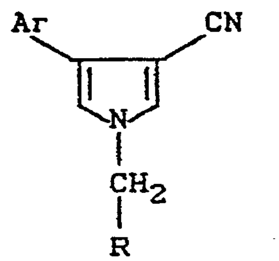

(I)

in which
Ar represents phenyl which is optionally monosubstituted to polysubstituted by identical or different substituents, suitable substituents being: halogen, in each case straight-chain or branched alkyl, alkoxy, alkylthio, halogenoalkyl, halogenoalkoxy or halogenoalkylthio in each case having 1 to 4 carbon atoms and, if appropriate, 1 to 9 identical or different halogen atoms, and R represents a

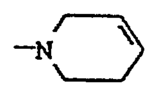 ; 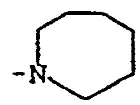 or

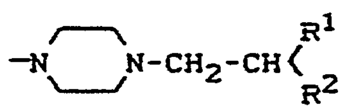 radical, where

$R^1$ represents hydrogen or straight-chain or branched alkyl having 1 to 6 carbon atoms and $R^2$ represents cyano or in each case straight-chain or branched alkanoyl, alkoxycarbonyl, alkylaminocarbonyl, dialkylaminocarbonyl, alkylsulphinyl or alkylsulphonyl in each case having 1 to 6 carbon atoms in the individual alkyl parts, or phenylsulphinyl or phenylsulphonyl.

2. 1-Aminomethyl-3-aryl-4-cyano-pyrroles according to Claim 1, where, in the formula (I),
Ar represents phenyl which is optionally monosubstituted, disubstituted of trisubstituted by identical of different substituents, suitable substituents being: fluorine, chlorine, bromine, methyl, ethyl, n- or i-propyl, n-, i-, s- or t-butyl, methoxy, ethoxy or methylthio, trifluoromethyl, trifluoromethoxy, or trifluoromethylthio,

R represents a 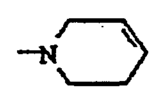 ; 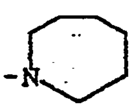 or

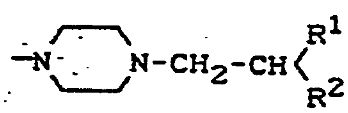 radical, where

$R^1$ represents hydrogen, methyl, ethyl, n- or i-propyl or n-, i-, s- or t-butyl and $R^2$ represents cyano, acetyl, propionyl, methoxycarbonyl, ethoxycarbonyl, methylaminocarbonyl, dimethylaminocarbonyl, ethylaminocarbonyl, diethylaminocarbonyl, dipropylaminocarbonyl, dibutylaminocarbonyl, methylsulphinyl, ethylsulphinyl, methylsulphonyl, ethylsulphonyl, phenylsulphinyl or phenylsulphonyl.

3. 1-Aminomethyl-3-aryl-4-cyano-pyrroles according to Claim 1, where, in the formula (I),
Ar represents a 2,3-dichlorophenyl radical, and

R represents a 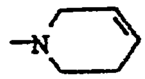 ; 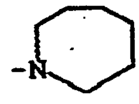 or

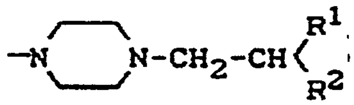 radical, where

$R^1$ represents hydrogen and
$R^2$ represents cyano, acetyl, propionyl, methoxycarbonyl, ethoxycarbonyl, dimethylaminocarbonyl, methylsulphinyl, methylsulphonyl, phenylsulphinyl or phenylsulphonyl.

4. Process for the preparation of 1-aminomethyl-3-aryl-4-cyano-pyrroles of the general formula (I)

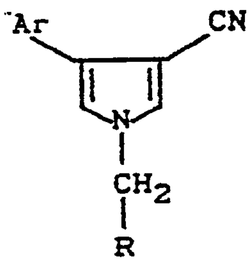 (I)

in which
Ar represents phenyl which is optionally monosubstituted to polysubstituted by identical or different substituents, suitable substituents being: halogen, in each case straight-chain or branched alkyl, alkoxy, alkylthio, halogenoalkyl, halogenoalkoxy or halogenoalkylthio is each case having 1 to 4 carbon atoms and, if appropriate, 1 to 9 identical or different halogen atoms, and R represents a

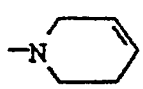 ; 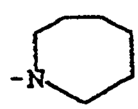 or

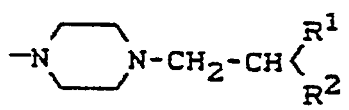 radical, where

$R^1$ represents hydrogen or straight-chain or branched alkyl having 1 to 6 carbon atoms and $R^2$ represents cyano or in each case straight-chain or branched alkanoyl, alkoxycarbonyl, alkylaminocarbonyl, dialkylaminocarbonyl, alkylsulphinyl or alkylsulphonyl in each case having 1 to 6 carbon atoms in the individual alkyl parts, or phenylsulphinyl or phenylsulphonyl, characterized in that

(a) 3-aryl-4-cyano-pyrroles of the formula (II)

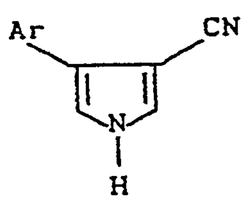 (II)

in which
Ar has the abovementioned meaning, are reacted with formaldehyde and amines of the formula (III)
R–H (III)
in which R has the abovementioned meaning, if appropriate in the presence of a diluent and if appropriate in the presence of a reaction auxiliary, or

(b) 3-aryl-4-cyano-pyrroles of the formula (IV)

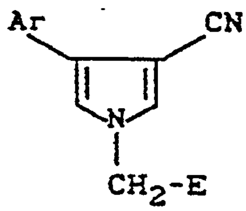

(IV)

in which
Ar has the a abovementioned meaning, and
E represents an electron-withdrawing leaving group, are reacted with amines of the formula (III)
H–R (III)
in which
R has the abovementioned meaning, if appropriate in the presence of a diluent and if appropriate in the presence of an acid-binding agent.

5. Pesticides, characterized in that they contain at least one 1-aminomethyl-3-aryl-4-cyano-pyrrole of the formula (I) according to Claims 1 and 4.

6. Use of 1-aminomethyl-3-aryl-4-cyano-pyrroles of the formula (I) according to Claims 1 and 4 for combating pests.

7. Process for combating pests, characterized in that 1-aminomethyl-3-aryl-4-cyano-pyrroles of the formula (I) according to Claims 1 and 4 are allowed to act on the pests and/or on their habitat.

8. Process for the preparation of pesticides, characterized in that 1-aminomethyl-3-aryl-4-cyano-pyrroles of the formula (I) according to Claims 1 and 4 are mixed with extenders and/or surface-active agents.

9. Use of 1-aminomethyl-3-aryl-4-cyano-pyrroles of the formula (I) according to Claim 5 for combating fungi.

**Revendications**

1. 1-aminométhyl-3-aryl-4-cyano-pyrroles de formule générale (I)

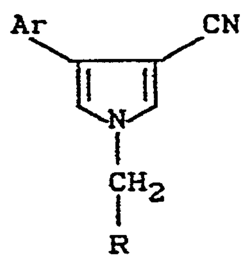

(I)

dans laquelle
Ar désigne un groupe phényle portant éventuellement un ou plusieurs substituants identiques ou différents, et on considère comme substituant: un halogène, un groupe alkyle, alkoxy, alkylthio, halogénalkyle, halogénalkoxy ou halogénalkylthio à chaîne droite ou ramifiée, ayant chacun 1 à 4 atomes de carbone et portant le cas échéant 1 à 9 atomes d'halogènes identiques ou différents et

R représente un reste 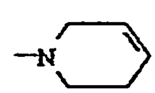 ; 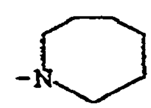 ou

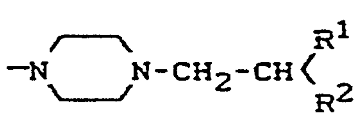

où
$R^1$ est l'hydrogène ou un groupe alkyle à chaîne droite ou ramifiée ayant 1 à 6 atomes de carbone et
$R^2$ est un groupe cyano ou un groupe alcanoyle, alkoxycarbonyle, alkylaminocarbonyle, dialkylaminocarbonyle, alkylsulfinyle ou alkylsulfonyle à chaîne droite ou ramifiée, ayant chacun 1 à 6 atomes de carbone dans les parties alkyle individuelles, ou un groupe phénylsulfinyle ou phénylsulfonyle.

2. 1-aminométhyl-3-aryl-4-cyano-pyrroles suivant la revendication 1, dans la formule (I) desquels
Ar est un groupe phényle portant éventuellement 1 à 3 substituants identiques ou différents, en considérant comme substituants: le fluor, le chlore, le brome, un groupe méthyle, éthyle, n-propyle, isopropyle, n-butyle, isobutyle, sec.-butyle, tertio-butyle, méthoxy, éthoxy ou méthylthio, trifluorométhyle, trifluorométhoxy, trifluorométhylthio et

R représente un reste 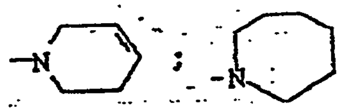 ou

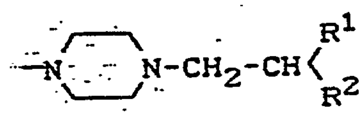

où

$R^1$ est l'hydrogène, un groupe méthyle, éthyle, n-propyle, isopropyle, n-butyle, isobutyle, sec.-butyle ou tertio-butyle et

$R^2$ est un groupe cyano, acétyle, propionyle, méthoxycarbonyle, éthoxycarbonyle, méthylaminocarbonyle, diméthylaminocarbonyle, éthylaminocarbonyle, diéthylaminocarbonyle, dipropylaminocarbonyle, dibutylaminocarbonyle, méthylsulfinyle, éthylsulfinyle, méthylsulfonyle, éthylsulfonyle, phénylsulfinyle ou phénylsulfonyle.

3. 1-aminométhyl-3-aryl-4-cyano-pyrroles suivant la revendication 1, dans la formule (I) desquels

Ar est un reste 2,3-dichlorophényle et

R est un reste 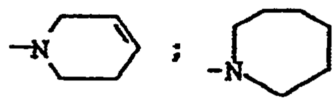 ou

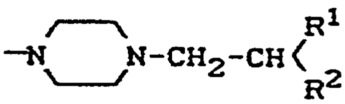

où

$R^1$ est l'hydrogène et

$R^2$ est un groupe cyano, acétyle, propionyle, méthoxycarbonyle, éthoxycarbonyle, diméthylaminocarbonyle, un groupe méthylsulfinyle, méthylsulfonyle, phénylsulfinyle ou phénylsulfonyle.

4. Procédé de production de 1-aminométhyl-3-aryl-4-cyano-pyrroles de formule générale (I)

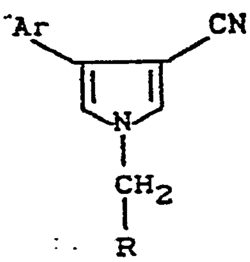 (I)

dans laquelle

Ar désigne un groupe phényle portant éventuellement un ou plusieurs substituants identiques ou différents, et on considère comme substituants: un halogène, un groupe alkyle, alkoxy, alkylthio, halogénalkyle, halogénalkoxy ou halogénalkylthio à chaîne droite ou ramifiée, ayant chacun 1 à 4 atomes de carbone et portant le cas échéant 1 à 9 atomes d'halogènes identiques ou différents et

R représente un reste

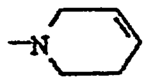 ; 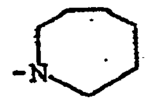 ou

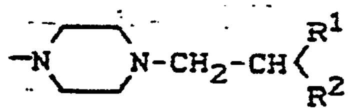

où
$R^1$ est l'hydrogène ou un groupe alkyle à chaîne droite ou ramifiée ayant 1 à 6 atomes de carbone et
$R^2$ est un groupe cyano ou un groupe alcanoyle, alkoxycarbonyle, alkylaminocarbonyle, dialkylaminocarbonyle, alkylsulfinyle ou alkylsulfonyle à chaîne droite ou ramifiée, ayant chacun 1 à 6 atomes de carbone dans les parties alkyle individuelles, ou un groupe phénylsulfinyle ou phénylsulfonyle, caractérisé en ce que

(a) on fait réagir des 3-aryl-4-cyanopyrroles de formule (II)

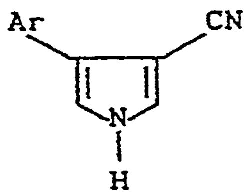

(II)

dans laquelle
Ar a la définition indiquée ci-dessus, avec le formaldéhyde et des amines de formule (III), R–H (III)
dans laquelle
R a la définition indiquée ci-dessus, le cas échéant en présence d'un diluant et en la présence éventuelle d'un adjuvant de réaction, ou bien

(b) on fait réagir des 3-aryl-4-cyano-pyrroles de formule (IV)

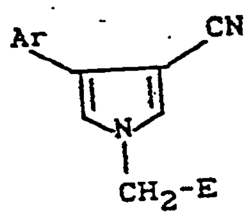

(IV)

dans laquelle
Ar a la définition indiquée ci-dessus et
E est un groupe partant attirant les électrons, avec des amines de formule (III)
H–R (III)
dans laquelle R a la définition indiquée ci-dessus, le cas échéant en présence d'un diluant et en la présence éventuelle d'un accepteur d'acide.

5. Compositions pesticides, caractérisées par une teneur en au moins un 1-aminométhyl-3-aryl-4-cyano-pyrrole de formule (I) suivant les revendications 1 et 4.

6. Utilisation de 1-aminométhyl-3-aryl-4-cyano-pyrroles de formule (I) suivant les revendications 1 et 4 pour combattre des pesticides.

7. Procédé pour combattre des parasites, caractérisé en ce qu'on fait agir des 1-aminométhyl-3-aryl-4-cyano-pyrroles de formule (I) suivant les revendications 1 et 4 sur des parasites et/ou sur leur milieu.

8. Procédé de préparation de compositions pesticides, caractérisé en ce qu'on mélange des 1-aminométhyl-3-aryl-4-cyano-pyrroles de formule (I) suivant les revendications 1 et 4 avec des diluants et/ou des agents tensio-actifs.

9. Utilisation de 1-aminométhyl-3-aryl-4-cyano-pyrroles de formule (I) suivant la revendication 5 pour combattre des champignons.